# EUROPEAN PATENT SPECIFICATION

(11) **EP 2 288 298 B1**
(45) Date of publication and mention of the grant of the patent: **29.02.2012**
(21) Application number: 09758991.5
(22) Date of filing: 20.05.2009
(51) Int. Cl.: A61B 17/16, A61B 17/17

(54) **SYSTEM AND TOOL FOR SURGICAL PROCEDURES**
SYSTEM UND WERKZEUG FÜR CHIRURGISCHE VERFAHREN
SYSTÈME ET OUTIL POUR DES INTERVENTIONS CHIRURGICALES

(30) Priority: 02.06.2008 US 131592
(43) Date of publication of application: 02.03.2011
(73) Proprietor: Warsaw Orthopedic, Inc., Warsaw, IN 46581 (US)
(72) Inventor: MIRE, David A., Cordova Tennessee 38018 (US); SALADINO, Joseph J., Memphis Tennessee 38119 (US)
(74) Representative: Viering, Jentschura & Partner
(86) International application number: PCT/US2009/044666
(87) International publication number: WO 2009/148830

(56) References cited:
- WO-A-2005/074831
- GB-A- 2 438 877
- US-A1- 2006 178 593
- US-A1- 2006 200 207
- US-A1- 2007 100 334

## Description

### BACKGROUND

The present invention relates generally to surgical tools and systems utilized in conjunction with implants, and more specifically, but not exclusively, to a surgical tool wirelessly connected to a neurological integrity monitoring system and/or a surgical navigation system.

Spinal implants can be engaged to or along one or more vertebrae of the spinal column for the treatment of various spinal conditions or pathologies. Fasteners can be provided to secure the implant to a particular location along the spinal column. The implants can be provided to stabilize the spinal column for treatment, either by fixing the spinal column or by permitting at least some motion of the stabilized motion segments.

Multi-axial and uni-axial screws have been employed for securing elongated implants, such as rods or plates, along one or more motion segments of the spinal column. The use of rod-to-bolt connectors to fix bone screws to spinal rods is another way to secure vertebrae together. Such fasteners can comprise many components or parts that make placement and manipulation of the fastener cumbersome during surgery. Securing the fasteners into position in the spine is generally a manual process including the use of powerless, hand-held tools.

US 2006/200207 A1 discloses a tissue stimulation system comprising a housing having proximal and distal ends, an operative element having an electrically conductive surface sized and configured for electrical stimulation of a targeted tissue region, the operative element extending from the distal end of the housing, and wherein the electrical stimulation is in the form of a signal having an amplitude and a duration for providing a first indication, a stimulation control device electrically coupled to the operative element, the stimulation control device comprising stimulation signal generating circuitry, the housing including a first control device for turning the stimulation signal to the operative element on and off and for providing adjustment of stimulation signal amplitude, the first control device being electrically coupled to the stimulation control device, and the housing including a second control device for providing adjustment of the stimulation signal duration, the second control device being electrically coupled to the stimulation control device.

US 2006/0178593 A1 discloses an apparatus comprising a tool portion configured to operate relative to bone tissue, wherein said tool portion includes an insulated shaft extending along a longitudinal axis and comprising an electrically conductive member with a non-insulated tip, wherein said shaft carries an electrical signal to said tip, a handle portion, removably and operably coupled to said tool portion, incorporating an operating system for operation of said tool portion, and a nerve monitoring system connected to said handle portion, wherein said nerve monitoring system is operable to detect a neural element threshold as a function of said electrical signal, and provide a signal to said operating system of said handle portion to change an operative mode of said tool portion upon detection of the neural element threshold.

GB 2 438 877 A discloses a control system for a portable power tool, said power tool including a motor configured to drive a cutting tool, the control system comprising a cutting tool for said power tool, said cutting tool having a sensor to sense a characteristic of an environment local to said cutting tool, a signal communications system to communicate a signal from said sensor to a controller, and a controller configured to output a control signal for controlling said power tool responsive to said signal from said sensor.

### SUMMARY

The invention provides a combination of a surgical tool with a neural integrity monitoring system according to claim 1. Further embodiments of the invention are described in the dependent claims.

A surgical tool is disclosed that is utilized during surgical procedures to implant a device, such as a bone screw, in a bone structure. The surgical tool includes a motor disposed within a housing coupled to an output shaft. A trigger mechanism is configured to selectively supply power to the motor to rotate the output shaft. A neural signal generation unit is configured to generate an expected electric signal that is provided to a surgical instrument connected with the output shaft. As such, the surgical instrument functions as a signal carrier for the expected electric signal.

The surgical tool is capable of being used in conjunction with a neural integrity monitoring system and/or a surgical navigation system. In one form, the neural integrity monitoring system and/or surgical navigation system is capable of conducting wireless communication with the surgical tool. The neural integrity monitoring system is operable to monitor the neurological status of one or more respective nerve elements of a patient. If a predetermined response is received by the neural integrity monitoring system, a wireless signal is sent to the surgical tool instructing the surgical tool to cease supplying power to the motor, thereby stopping the motor from rotating the surgical instrument. If the surgical navigation system detects that the implant is about to breach a safe threshold or has breached a safe threshold, a wireless signal is sent to the surgical tool to cease supplying power to the motor, thereby once again stopping the motor from rotating the surgical instrument. After a predetermined amount of time, the surgical tool can reset itself to once again operate normally so that the operator can take appropriate action or, in the alternative, the operator can use a reset located on either the neural integrity monitoring system or surgical navigation device to reset the surgical tool.

The surgical tool also includes at least one optical indicator located on an outside surface of the housing. The optical indicator is configured to display the neurological status of the patient. If no response is detected by the neural integrity monitoring system, then the neural integrity monitoring system generates a wireless signal that causes the surgical tool to illuminate the optical indicator in a predetermined color, which in one form is green for example. If a response is detected by the neural integrity monitoring system that goes above a specified neural threshold, then the neural integrity monitoring system generates a wireless signal that causes the surgical tool to illuminate the optical indicator in a different predetermined color, which in one form is red for example. Likewise, if a safe threshold defined on an image of the patient is about to be breached or has been breached, the surgical navigation system is configured to generate a wireless signal that causes the surgical instrument to switch the optical indicator from a safe indication state (e.g. - green) to a warning indication state (e.g. - red). This provides the operator with a visual indication on the surgical tool to allow the operator to readily determine the neurological status of the patient and/or the positional status of an implant in relation to a predefined safe threshold.

The surgical tool further includes an audible indicator, such as a speaker or transducer, which is capable of generating audible alarms. In one form, if the neurological status of the patient is at risk, the neural integrity monitoring system transmits a wireless signal to the surgical tool that causes the surgical tool to generate an audible alarm warning the operator of the impending risk. In another form, the surgical tool is capable of operating in a nerve proximity mode by generating audible sounds that increase in intensity as a function of the response detected by the neural integrity monitoring system. As such, the closer the implant device or surgical instrument get to a neural element, the louder and faster the audible warning will be on the surgical tool to provide the operator with a proximity detection feature. The surgical navigation system works in much the same manner, except in this case the operator is provided with audible warnings that increase in intensity the closer the surgical instrument or implant device gets to the predefined safe threshold.

The following method may be applied for implanting an implant device in a patient. The method includes attaching a neural integrity monitoring system to the patient to monitor a neurological status of the patient during a surgical procedure; providing a surgical tool including a motor connected with a trigger mechanism for selective operation of the motor; using a surgical instrument connected with an output shaft of the surgical tool during the surgical procedure to assist in implantation of a device in the patient; generating an electric signal that is electrically transmitted to the surgical instrument at least while the trigger mechanism is depressed to activate the motor; and stopping the surgical tool from rotating the surgical instrument if a response above a specified threshold is detected by the neurological integrity monitoring system.

The following further method may be applied for implanting an implant device in a patient. This method includes generating an anatomical image of a portion of the patient undergoing a surgical procedure with a surgical navigation system; defining a safe threshold on the anatomical image of the portion of the patient for implantation of a device; providing a surgical tool including a motor connected with a trigger mechanism for selective operation of the motor; using a surgical instrument attached to the surgical tool during the surgical procedure to assist in implantation of the device in the patient; and automatically stopping the surgical tool from rotating the surgical instrument if the safe threshold is about to be breached by the device.

Further, additional features and advantages are realized through the techniques of the present invention. Other embodiments and aspects of the invention are described in detail herein and are considered a part of the claimed invention.

### BRIEF DESCRIPTION OF THE FIGURES

The figures are not necessarily to scale, emphasis instead being placed upon illustrating the principles of the invention. Moreover, in the figures, like reference numerals designate corresponding parts throughout the different views and forms.
Fig. 1 depicts a lateral view of a portion of a vertebral column.
Fig. 2 depicts a top plan view of a vertebra.
Fig. 3 depicts a cross-section illustration of an illustrative surgical tool.
Fig. 4 depicts a cross-section illustration of a portion of a surgical tool.
Fig. 5 depicts a perspective view of a portion of a surgical tool.
Fig. 6A depicts a perspective view of a direction selection assembly of a surgical tool.
Fig. 6B depicts a perspective view of an anti-backdiive assembly and a direction selection assembly of a surgical tool.
Fig. 7 depicts a side view of a representative surgical tool.
Fig. 8 depicts a side view of a portion of a representative surgical tool that includes a representative NIM member.
Fig. 9 depicts a side view of a representative surgical tool that includes another representative NIM member.
Fig. 10 depicts a cross-sectional view of a surgical tool including a representative NIM member.
Fig. 11 depicts a cross-sectional view of a surgical tool including yet another representative NIM member.
Fig. 12 depicts a cross-sectional view of a surgical tool including a navigational enabling member.
Fig. 13 depicts a system that integrates a representative surgical tool with a neural integrity monitoring system.
Fig. 14 depicts a system that integrates a representative surgical tool with a surgical navigation system.
Fig. 15 illustrates a representative graphical user interface of the NAV software application depicting a representative radiographic image generated on a display.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended. Any such alterations and further modifications in the illustrated devices and described methods, and any such further applications of the principles of the invention as illustrated herein are contemplated as would normally occur to one skilled in the art to which the invention relates.

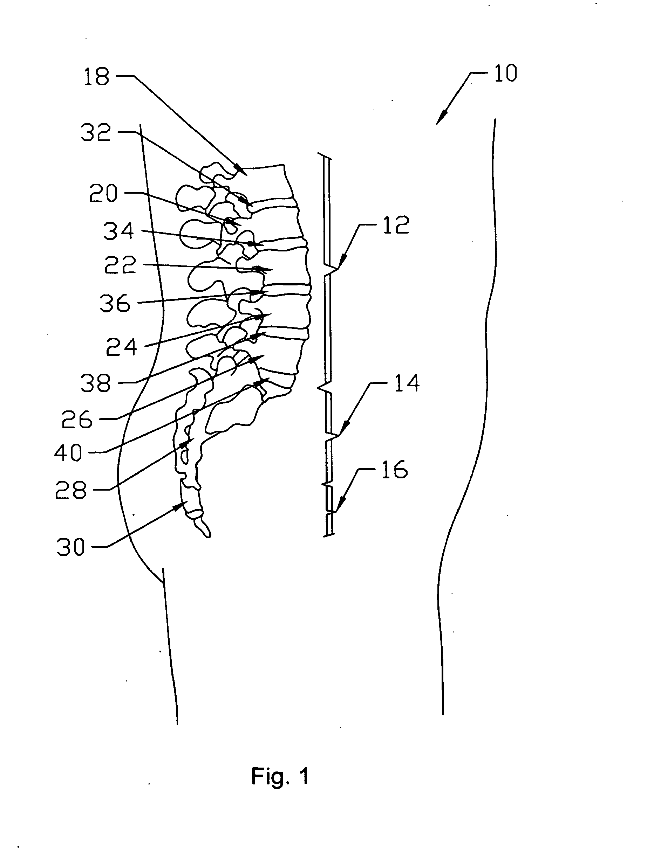

Referring to Fig. 1, a portion of a vertebral column, designated 10, is shown. As depicted, vertebral column 10 includes a lumbar region 12, a sacral region 14, and a coccygeal region 16. The vertebral column 10 also includes a cervical region and a thoracic region. For clarity and ease of discussion, the cervical region and the thoracic region are not illustrated. Lumbar region 12 includes a first lumbar vertebra 18, a second lumbar vertebra 20, a third lumbar vertebra 22, a fourth lumbar vertebra 24, and a fifth lumbar vertebra 26. The sacral region 14 includes a sacrum 28. Further, the coccygeal region 16 includes a coccyx 30.

As depicted in Fig. 1, a first intervertebral lumbar disc 32 is disposed between first lumbar vertebra 18 and second lumbar vertebra 20. A second intervertebral lumbar disc 34 is disposed between second lumbar vertebra 20 and third lumbar vertebra 22. A third intervertebral lumbar disc 36 is disposed between third lumbar vertebra 22 and fourth lumbar vertebra 24. Further, a fourth intervertebral lumbar disc 38 is disposed between fourth lumbar vertebra 24 and fifth lumbar vertebra 26. Additionally, a fifth intervertebral lumbar disc 40 is disposed between fifth lumbar vertebra 26 and sacrum 28.

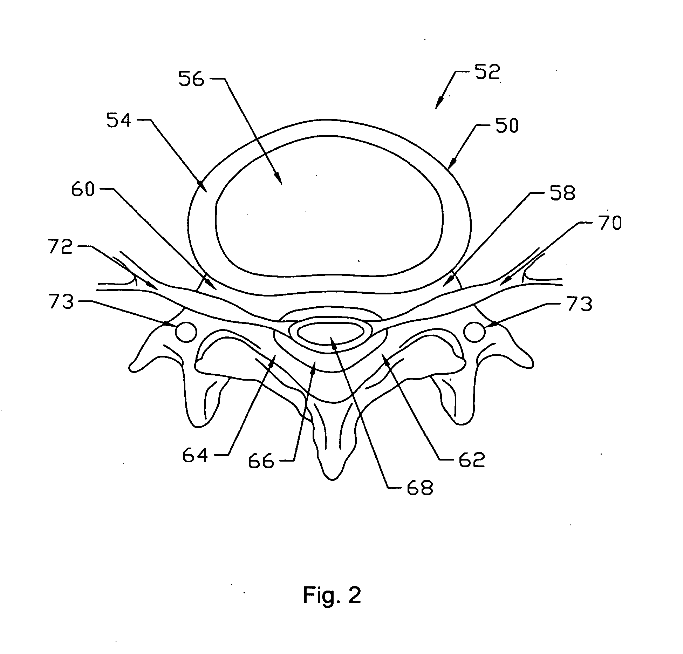

Referring to Fig. 2, a top plan view of a respective vertebral is illustrated. As shown, a vertebral body 50 of an inferior vertebra 52 includes a cortical rim 54 composed of cortical bone. Also, vertebral body 50 includes cancellous bone 56 within cortical rim 54. Cortical rim 54 is often preferred to as the apophyseal rim or apophyseal ring. Further, cancellous bone 56 is generally softer than the cortical bone of the cortical rim 54.

As illustrated in Fig. 2, the inferior vertebra 52 further includes a first pedicle 58, a second pedicle 60, a first lamina 62, and a second lamina 64. Further, a vertebral foramen 66 is established within inferior vertebra 52. A spinal cord 68 passes through vertebral foramen 66. Moreover, a first nerve root 70 and a second nerve root 72 extend from spinal cord 68. In particular, first pedicle 58 and second pedicle 60 represent regions of the spine in which surgeons often choose to implant anchors 73, such as bone screws for attaching an anchor and rod system to the spine. Notably, given the proximity to spinal cord 68 and other significant anatomical portions, the implantation of such screws is a delicate and precise procedure requiring tools significantly different than available to the general public.

The vertebrae that make up the vertebral column have slightly different appearances as they range from the cervical region to the lumbar region of the vertebral column. However, all vertebras, except the first and second cervical vertebrae, have the same basic structures, e.g., those structures described above in conjunction with Fig. 2. The first and second cervical vertebrae are structurally different than the rest of the vertebrae in order to support a skull.

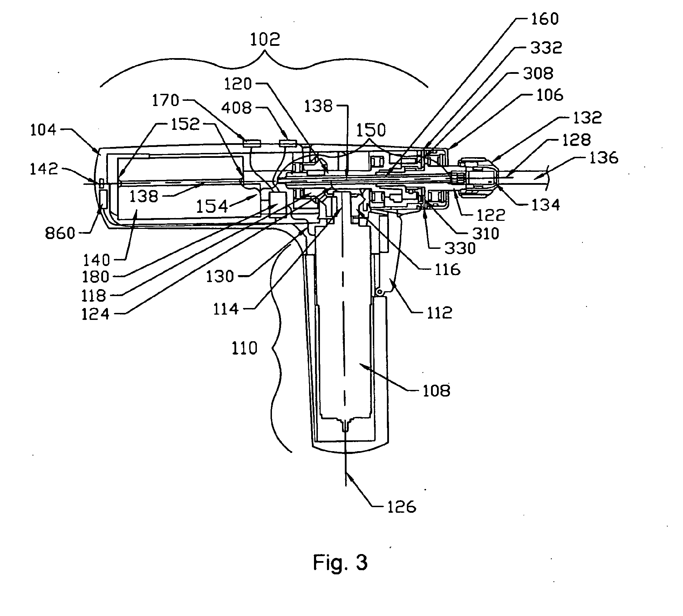

Figs. 3-19 provide illustrations of a surgical tool 100 or portions of surgical tool 100 in accordance with the present application. In particular, Figs. 3-19 illustrate a surgical tool 100 configured for affixing orthopedic anchors, intervertebral bodies such as threaded cages, and screws in bone or spaces between bones, such as between vertebra. Surgical tool 100 is suitable for tapping bone, including using a tapper bit head to form a pilot hole within the bone. Moreover, surgical tool 100 is particularly well suited for driving a screw into bone using a screwdriver bit head. Surgical tool 100 facilitates initial driving of a screw into bone using the power of surgical tool 100, and then optionally, manually finishing the driving process of setting the screw using surgical tool 100 as it has improved feel and ratcheting capabilities.

Referring to Fig. 3, a cross-sectional illustration of surgical tool 100 is illustrated that includes a housing 102 extending from a proximal end 104 to a distal end 106 opposite proximal end 104. In general reference to the operation of surgical tool 100, housing 102 includes a motor 108 disposed within a handle 110 and coupled to housing 102. A trigger mechanism 112 is coupled to handle 100 and is electrically coupled to motor 108 such that upon depression of trigger mechanism 112 by a user, motor 108 is engaged to rotate a surgical instrument 136. Surgical instrument 136 may comprise a drill, a tap, a probe, a drive mechanism, such as a socket, screwdriver, keyed driver (e.g. - hex head drive, star head driver, square head driver, etc.), an instrument 136 and implant 73 combination, an implant 73 that is capable of detachably connecting to surgical tool 100, or any other type of surgical instrument that requires rotation during a surgical procedure.

Motor 108 includes a drive shaft 114 extending from motor 108 and coupled to a transfer mechanism 116. In one form, transfer mechanism 116 includes a bevel gear. Additionally, in another form, transfer mechanism 116 can be coupled to a second transfer mechanism 118, which can include a second bevel gear, which in turn is coupled to an output shaft 120. Accordingly, engagement of trigger mechanism 112 can include engagement of motor 108 to turn transfer mechanism 116, and correspondingly rotate second transfer mechanism 118, thereby causing rotation of output shaft 120. Accordingly, a surgical tool 100 is provided that is capable of providing a rotational force to a work piece.

Additionally as will be illustrated in other forms, output shaft 120 can be separated into various sections including a proximal output shaft portion 120, which is coupled to a distal output shaft portion 122. Housing 102 is connected to handle 110 extending at an angle from housing 102 and coupled to housing 102 between proximal end 104 and distal end 106. In one form, handle 110 extends from housing 102 at a substantially orthogonal angle 124 as defined between an axis 126 of handle 110 relative to an axis 128 of housing 102. In accordance with another form, angle 124 can be a non-orthogonal angle such that handle 110 is angled relative to housing 102. As such, in forms utilizing angle 124 that is substantially non-orthogonal, handle 110 has a forward rake design, such that a proximal end 130 of handle 110 is angled toward distal end 106 of housing 102.

Surgical tool 100 can include a movable chuck 132 coupled to housing 102 at distal end 106. According to one form, chuck 132 can include an opening 134 configured to engage a shaft of surgical instrument 136. In yet another form, chuck 132 can include a quick-connect adapter, configured to engage a proximal end of surgical instrument 136 via a snap-fit connection. As will be appreciated, such a quick-connect adapter can include biasing members or channels for receiving bearings to accomplish the snap-fit or quick-connect coupling. In one representative form, surgical tool 100 includes a passage 138 extending through housing 102 from distal end 106 to proximal end 104. As illustrated, passage 138 can extend through the interior of output shaft 120 and through the interior of a battery pack 140 and exit housing 102 at an opening 142 adjacent to proximal end 104. Passage 138 can have a generally circular cross-sectional contour having a predetermined diameter.

In one for, passage 138 is segmented and includes two discrete passage portions including a first portion 150 disposed within the interior of output shaft 120 and a second portion 152 extending through the interior of battery pack 140, wherein first portion 150 and second portion 152 are axially separated by an opening 154. While passage 138 can be segmented including a first portion 150 and a second portion 152, it will be appreciated that passage 138 can be further segmented into three portions, or even more portions. Still according to another form, passage 138 can be a single, sealed passage extending for the entire length of housing 102 between the proximal end 104 and distal end 106 without any openings. Still, according to other embodiments, the passage can have alternative designs. For example, in one form passage 138 can be a channel including an opening that extends substantially along the longitudinal length of passage 138. In another form passage 138 can include a series of openings or perforations extending along the length of passage 138.

In accordance with another form, passage 138 can include an electrically insulated liner 160 extending along the interior surface of passage 138 and extending for a portion of passage 138. Electrically insulating liner 160 can facilitate electrical insulation between passage 138 and surrounding components contained within housing 102. Electrically insulated liner 160 can extend for only a portion of the length of passage 138. In reference to forms wherein passage 138 is segmented, one of the portions can include electrically insulating liner 160 while the second portion may not include electrically insulating liner 160. For example, as illustrated in Fig. 3, first portion 150 includes an electrically insulating portion 160 while second portion 152 of passage 138 does not include electrically insulating liner 160. However, it is particularly suitable that at least a portion of passage 138 extending through battery pack 140 include electrically insulating liner 160.

Materials suitable for forming the electrically insulating lining 160 can generally include dielectric materials. Various dielectric materials can include ceramics or polymers. According to a particular form, electrically insulating lining 160 includes a polymer material. In one form, suitable polymer materials can include polyurethane materials, polyolefin materials, polyether materials, silicone materials, or a combination thereof. Further, the polyolefin materials can include polypropylene, polyethylene, halogenated polyolefin, fluoropolyolefin, or a combination thereof. The polyether materials can include polyetherketone (PEK), polyetheretherketone (PEEK), polyetherketoneketone (PEKK), polyaryletherketone (PAEK), or a combination thereof.

In further reference to sterilization of surgical tool 100, housing 102 and components contained therein can be made of autoclavable materials. As used herein, reference to autoclavable materials include materials capable of withstanding temperatures in excess of 121° C and pressures in excess of 15 PSIA. As such, in one particular form, suitable autoclavable materials can include metal, metal alloys, or polymers. According to one form, housing 102 and components contained within housing 102 can include metals such as titanium, aluminum, magnesium, iron, cobalt, nickel, tungsten, steel, or any combination thereof. In a more particular form, certain components can be made of polymer materials, including for example polyurethane materials, polyolefin materials, polyether materials, silicone materials, or a combination thereof. Further, the polyolefin materials can include polypropylene, polyethylene, halogenated polyolefin, fluoropolyolefin, or a combination thereof. The polyether materials can include a polyetherketone (PEK), polyetheretherketone (PEEK), polyetherketoneketone (PEKK), polyaryletherketone (PAEK), or a combination thereof. Other suitable materials can include styrenes (e.g., acrylonitrile butadiene styrene), polycarbonates, polysulphones, and carbon fiber, for example, carbon fiber-reinforced composites.

In reference to another for, portions of housing 102 can include electromagnetic shielding. In one form, the electromagnetic shielding can include metal meshing disposed around particular components within housing 102. Battery pack 140 and motor 108 can include electromagnetic shielding provided around at least a portion of the outer surface, and more particularly substantially surround the outer surfaces. Electromagnetic shielding of the components, particularly of battery pack 140 and motor 108, facilitates efficient and reliable use of surgical tool 100 in an environmental such as an operating room wherein many electrical machines are used and electromagnetic interferences may occur.

In reference to another particular form, surgical tool 100 can further include a torque limiter 170, which may be selectable by the user. Acco ding to one form, surgical tool 100 includes a control unit 180, which may comprise a microprocessor having memory, electrically coupled to motor 108 and battery pack 140 wherein microprocessor 180 controls the current from battery 140 to motor 108, thereby controlling the torque output of motor 108. Torque limiter 170 is connected with control unit 180 and allows variable adjustment of the torque output of motor 108. In accordance with an alternative form, surgical tool 100 can include a mechanical torque limiter coupled to motor 108 and output shaft 120. One such suitable torque limiter can include bearings and a clutch wherein if a particular torque is exceeded a first portion of output shaft 120 can be decoupled from a second portion of output shaft 120.

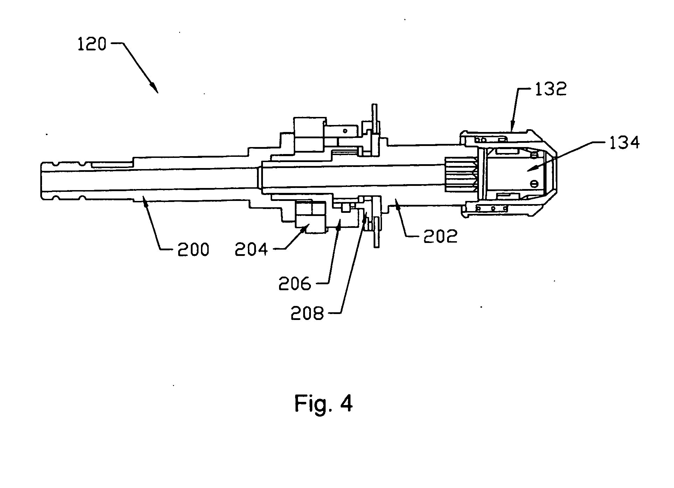

Referring to Fig. 4, a cross-sectional illustration of output shaft 120 of surgical tool 100 is provided in accordance with a representative form. As illustrated, output shaft 120 is segmented such that it has a proximal portion 200 coupled to a distal portion 202. In accordance with a particular embodiment, proximal portion 200 of output shaft 120 is coupled to distal portion 202 via a jam ring 204, which is coupled to a ratcheting mechanism 206, which in turn is coupled to a member 208. The combination of these components will be described in further detail in subsequent figures and the combination of these components (i.e., 204, 206 and 208) facilitate ratcheted rotation of output shaft 120 such that rotational movement of output shaft 120 can be finely controlled, which is particularly suitable for surgical procedures.

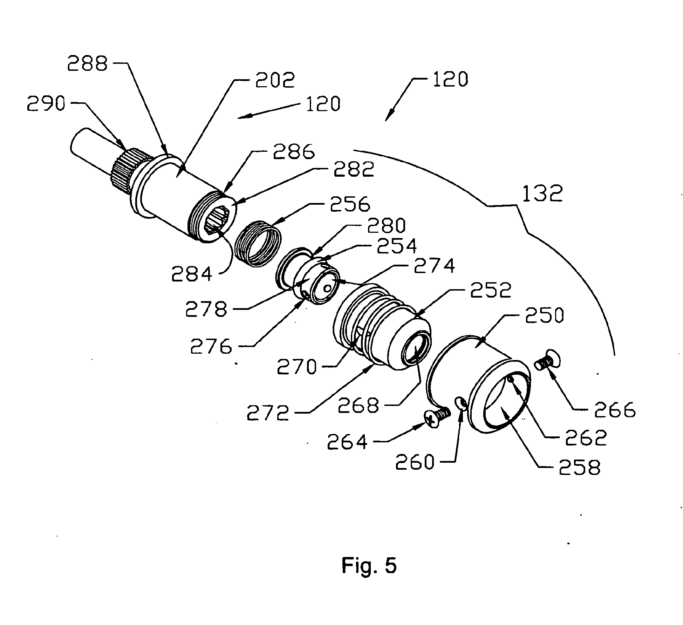

Fig. 5 includes a perspective view of output shaft 1.20 as previously illustrated in Fig. 4. Referring to Fig 5, a perspective view of the distal portion of output shaft 120 of surgical tool 100 is provided in accordance with a representative form. As illustrated, surgical tool 100 includes chuck 132 having an outer sleeve portion 250 coupleable to an intermediate sleeve portion 252, which is coupleable to an inner sleeve portion 254, which in turn is coupleable to a biasing member 256. Outer sleeve portion 250 provides the outer surface of chuck 132 and houses sleeve portion 252 and inner sleeve portion 254. As illustrated, outer sleeve portion 250 can include an opening 258 configured to allow extension of sleeve portion 252 therethrough for engagement of surgical instrument 136. Outer sleeve portion 250 includes openings 260 and 262 configured to engage screws 264 and 266 that are configured to couple the outer sleeve portion 250, sleeve portion 252, and inner sleeve portion 254.

Sleeve portion 252 of chuck 132 includes an opening 268 configured to extend through opening 258 of outer sleeve portion 250 and engage a surgical instrument 136. Sleeve portion 252 is configured to slide within and engage a portion of the inner surface of outer sleeve portion 250. Moreover, sleeve portion 252 includes an opening 270 axially located along the length of sleeve portion 252 and configured to engage with screws 264, 266 thereby coupling sleeve portion 252 with outer sleeve portion 250. Moreover, sleeve portion 252 can include a biasing member 272, such as a coiled spring, configured to engage an inner surface of outer sleeve portion 250 and bias outer sleeve portion 250 against sleeve portion 252.

Chuck 132 further includes inner sleeve portion 254 configured to engage the inner surface to sleeve portion 252. According to the illustrated embodiment, the inner sleeve portion 254 includes an opening 274 configured to align with opening 268 to sleeve portion 254, opening 258 of outer sleeve portion 250, and configured to receive surgical instrument 136. According to a particular form, inner sleeve portion 254 includes openings 276 displaced circumferentially around a collar portion 278 of the inner sleeve along surgical instrument 136 for a quick-connect coupling. Additionally, inner sleeve portion 254 includes a channel region 280 extending circumferentially around the outer surface of inner sleeve portion 254 and configured to engage with screws 264, 266 thereby fixably attaching inner sleeve portion 254 within sleeve portion 252 and further within outer sleeve portion 250.

Chuck 132 further includes a biasing member 256, such as a coiled spring, configured to couple between a front surface of distal portion 202 of output shaft 120 and a back surface of inner sleeve portion 254. Biasing member 256 facilitates decoupling of a bit shaft from chuck 132 by compressing inner sleeve portion 254 axially toward a face portion 282 of distal portion 202 of output shaft 120 thereby enabling one-handed decoupling of surgical instrument 136.

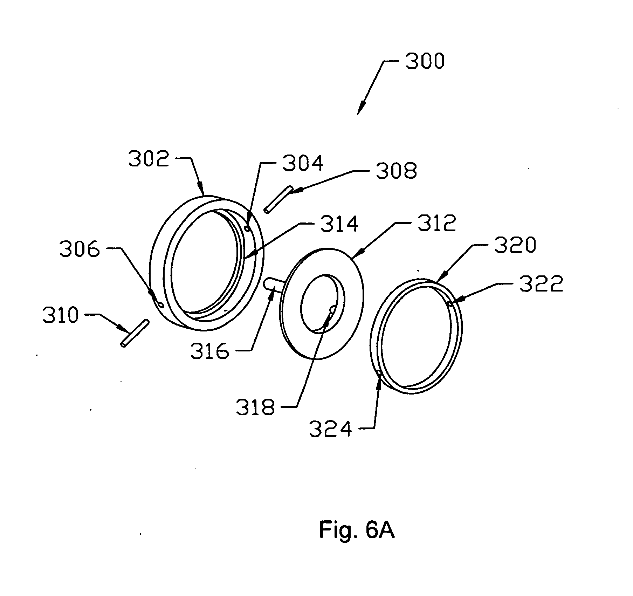

Distal portion 202 of output shaft 120 includes an opening 284 configured to receive a portion of surgical instrument 136. As illustrated, opening 284 can have a cross-sectional contour configured to engage a proximal end of a bit shaft, such as having a hexagonal cross-sectional contour as illustrated. Moreover, distal portion 202 of output shaft 120 includes threading 286 configured to engage threads within an inner surface of inner sleeve portion 252 and fixably couple chuck 132 on the end of distal portion 202 of output shaft 120. According to another form, distal portion 202 of output shaft 120 further includes a lip 288 configured to engage and directly contact components further illustrated in Fig. 6A.

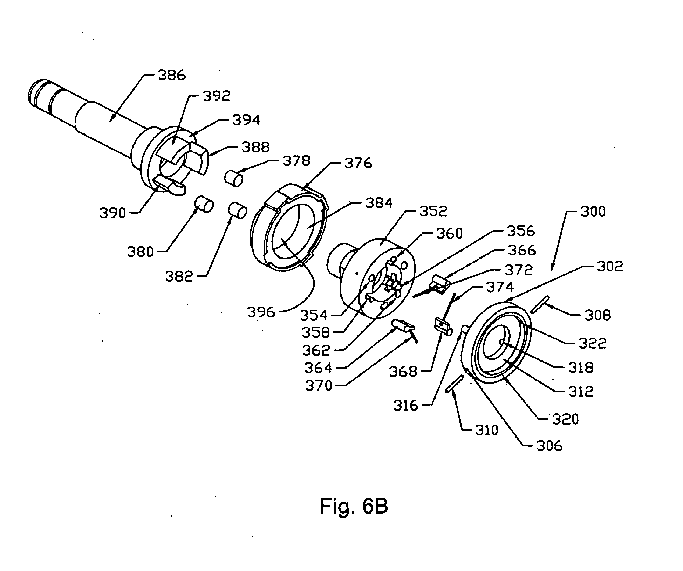

Distal portion 202 of output shaft 120 can include a toothed surface 290 extending around the circumference of the outer surface and configured to engage components further illustrated in Fig. 6B. In particular, teeth 290 facilitate coupling of a ratcheting mechanism (see Fig. 6B) for fine control. As such, in one form, toothed surface 290 includes at least twenty (20) total teeth extending around the circumference of distal portion 202. In a more particular form, toothed surface 290 includes between about thirty to fifty (30-50) total teeth on toothed surface 290. The provision of toothed surface 290 having such numbers of total teeth facilitates fine ratcheting control, which is particularly suitable for use during surgical procedures.

Figs. 6A and 6B include perspective views of a portion of output shaft 120 for use in surgical tool 100 in accordance with a representative form. Generally, Fig. 6A illustrates a direction selection assembly 300 used to select between a forward and reverse drive position. Fig. 6B generally illustrates components of the ratcheting mechanism and an anti-backdrive assembly. Referring to Fig. 6A, a sleeve 302 is provided having openings 304 and 306 configured to engage pins 308 and 310. The direction selection assembly 300 further includes a member 312 configured to be disposed within sleeve 302 such that it abuts inner lip 314 of sleeve 302. Member 312 further includes one or more protrusions or pins extending from a rear surface and configured to engage portions of an anti-backdrive device illustrated in Fig. 6B. In one form, a pin 316 of member 312 is configured to engage openings within the anti-back drive device, thereby acting as a clutch and capable of locking pawls (illustrated in Fig. 6B) within the anti-backdrive device in a particular location.

Member 312 includes at least one pin 318 that is shorter in length than pin 316 and particularly configured to selectively engage openings within the anti-backdrive device to engage or disengage the ratcheting capabilities. In accordance with one particular embodiment, member 312 includes three pins having the same length of pin 316 and three pins having a shorter length, such as pin 318 extending from the back surface of member 312.

As further illustrated in Fig. 6A, the direction selection assembly 300 further includes a sleeve 320 having openings 322 and 324 extending through the width of the body. Sleeve 320 is configured to fit within the inner diameter of sleeve 302 and hold member 312 within sleeve 302. In accordance with one form, while member 312 is within sleeve 302 it is generally free to rotate. Moreover, openings 322, 324 are configured to engage pins 308, 310 such that pins 308, 310 extend through openings 322, 324 and are flush with the inner surface of sleeve 320.

In accordance with one form, pins 308, 310 are configured to engage channels or slots (330 and 332 in Fig. 3) along the inner surface of housing 102. Pins 308, 310 can freely slide within the channels and upon movement of pins 308, 310 from one position to another, pin 318 of member 312 can engage or disengage an opening on the anti-backdrive device and accordingly select or deselect actuation of the ratcheting mechanism. In accordance with another form, pins 308, 310 can be engaged within cam slots inside a cap that is keyed to housing 102 to facilitate direction selection control.

Fig. 6B includes a perspective view of the components of an anti-backdrive assembly 350 and the direction selection assembly 300. As illustrated, the anti-backdrive assembly 350 is configured to engage direction selection assembly 300. Anti-backdrive assembly 350 includes anti-backdrive device 352, which includes openings 354 and 356 configured to engage pins 316, 318, respectively of direction selection assembly 300 in certain situations. Anti-backdiive device 352 further includes openings 358, 360, and 362 for housing pawls 364, 366, and 368 and corresponding biasing members 370, 372, and 374 respectively. According to a particular form, the ratcheting action of anti-back drive device 352 is facilitated by pawls 364, 366, and 368 configured to be disposed within openings 358, 360, and 362, respectively. Additionally, in one particular form, pawls 364-368 are resiliently biased against a portion of anti-backdrive device 352 such that they extend into the opening and are configured to engage teeth (previously illustrated as teeth 290 in Fig. 5) by biasing members 370, 372, and 374.

As further illustrated in Fig. 6B, anti-backdrive assembly 350 further includes a jam ring 376 configured to engage a surface of anti-backdrive device 352. Moreover, as further illustrated, rollers 378, 380, and 382 are configured to be disposed and coupled with an inner surface 384 of jam ring 376. In one form, a proximal portion of an output shaft 386 includes arms 388, 390, and 392 axially extending from a distal surface 394 which are configured to extend into an opening 396 of the jam ring 376 and engage a portion of the anti-back drive device when motor 108 is providing torque to the proximal portion of output shaft 386. The coupling between arms 388-394 with portions of anti-backdrive device 352 facilitates transfer of torque from the proximal portion of output shaft 386 to the distal portion of output shaft 202 (see Fig. 5) when motor 108 is providing the torque. Alternatively, if torque is applied to the proximal portion from the distal portion of output shaft 202 (i.e., not from the motor), jam ring 376 facilitates decoupling of motor 108 from output shaft 202 to avoid damage to motor 108. In particular, in situations where torque is applied to the portion from the opposite end of motor 108, rollers 378-382 lock against the inner surface of jam ring 376 and decouple the distal portion of output shaft 202 from the proximal portion.

In operating surgical tool 100 in a forward direction under the power of motor 108, pins 308, 310 move in slots or channels within the housing and pin 318 of the member 312 is disengaged from opening 356 in anti-backdrive device 352. The proximal portion of output shaft 386 rotates clockwise and arms 390-394 engage and contact surfaces of anti-backdrive device 352 while the rollers 628-630 spin freely within inner surface 384 of jam ling 376. The rotational motion is imparted to the distal portion of the output shaft 202 and it rotates while pawls 364-368 engage the teeth of toothed surface 290 providing torque transfer.

Surgical tool 100 can be operated in a forward direction under manual power up to a specific torque, which may be selected by the user or pre-set by the manufacturer upon assembly. During forward manual operation, if the specific torque is exceeded output shaft 386 can be rotated in reverse or backdrive. Upon exceeding the specified torque, the proximal portion of output shaft 386 can reverse rotation for a short distance until the arms 390-394 and rollers 378-382 lock up in inner surface 384 of jam ring 376. Since, according to one form, the jam ring 376 is locked to housing 102 of tool 100, the backdrive is stopped and manual forward driving of the output shaft can continue. It should be noted, that in this instance, pawls 364-368 may freely engage the teeth of the toothed surface 290 as they normally would when surgical tool 100 is operated in the forward direction under power.

Surgical tool 100 can be operated in a reverse direction, either using motor 108 or manual power. Using the direction selection assembly, and particularly changing the position of pins 308, 310 in the channels within the housing can select the reverse direction. Upon changing the position of pins 308, 310, pin 318 of member 312 is engaged within opening 356 of anti-backdrive device 352, thereby locking pawls 364-368 in a fixed or engaged position with toothed surface 290 and not allowing them to "flip". Engagement of pin 318 within opening 356 removes the ratcheting action. Accordingly, in one form, upon operation of surgical tool 100 in reverse under power, arms 388-392 transfer torque directly to pin 318 of anti-backdiive device 352 via engagement of pawls 364-368 with pin 318. As such, in accordance with a particular form, there is no ratcheting function in the reverse direction.

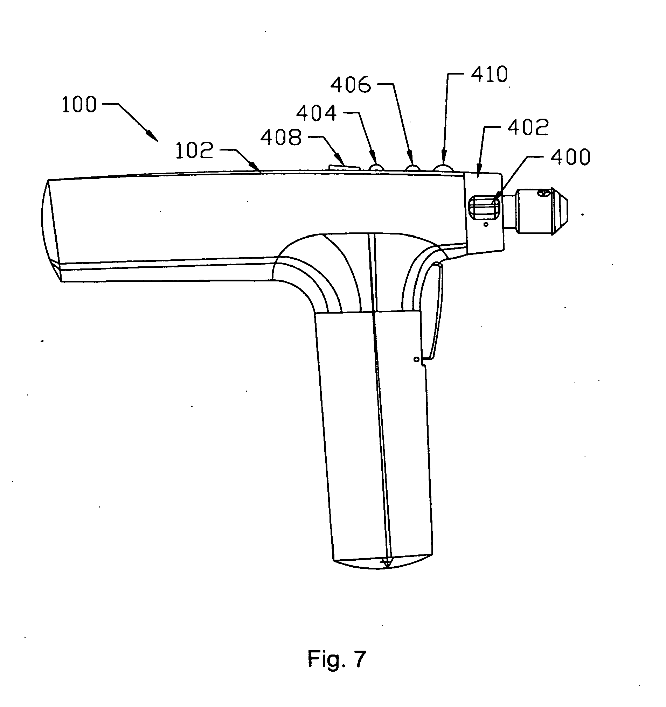

Fig. 7 illustrates a side view of surgical tool 100 in accordance with a representative form. Surgical tool 100 can include a switch 400 coupled to housing 102 and electrically coupled to motor 108. Switch 400 is moveable between a first position and a second position corresponding to a forward operating position and a reverse operating position of motor 108. In another particular form, switch 400 is coupled to a collar 402, both of which are moveable such as around the circumference of housing 102 to select between a forward operating condition and a reverse operating condition of motor 108. In accordance with one particular form, electrical coupling of switch 400 with motor 108 can include ganging of a forward electrical switch and a reverse electrical switch, such that both must be operated to change the direction of motor 108. Such a combination can facilitate certain safety control that may be suitable for use in a surgical tool.

In accordance with another form, switch 400 can further include a neutral position. Generally, the neutral position of switch 400 may include decoupling of power to motor 108, thereby making surgical tool 100 suitable for a manual ratcheting procedure. In accordance with another form, the switch 400 can include a neutral position wherein the output shaft is disengaged from the ratcheting position.

Surgical tool 100 can further include a first optical indictor 404 for indicating a state of surgical tool 100 to the user. For example, in one particular form optical indicator 404 can be electrically connected to the tools power source (i.e. battery pack 140) and electrically connected to switch 400 and configured to indicate a forward or reverse state of motor 108. In accordance with a particular form, optical indicator 404 can display an output when switch 400 has selected motor 108 to rotate in a forward direction, and a second optical indicator 406 can display an output when switch 400 has selected motor 108 to rotate in a reverse direction. Suitable optical indicators can include lights such as LED's or the like. More particularly, optical indicators 404, 406 can have different optical qualities, such as color, making it suitable for the operator to verify the position of switch 400 and the driving direction of motor 108. As will be appreciated, in forms utilizing switch 400 having a neutral position, an optical indicator may be coupled to that position of switch 400 as well, or alternatively, neither of the optical indicators 404, 406 may display an output when motor 108 is switched to the neutral position.

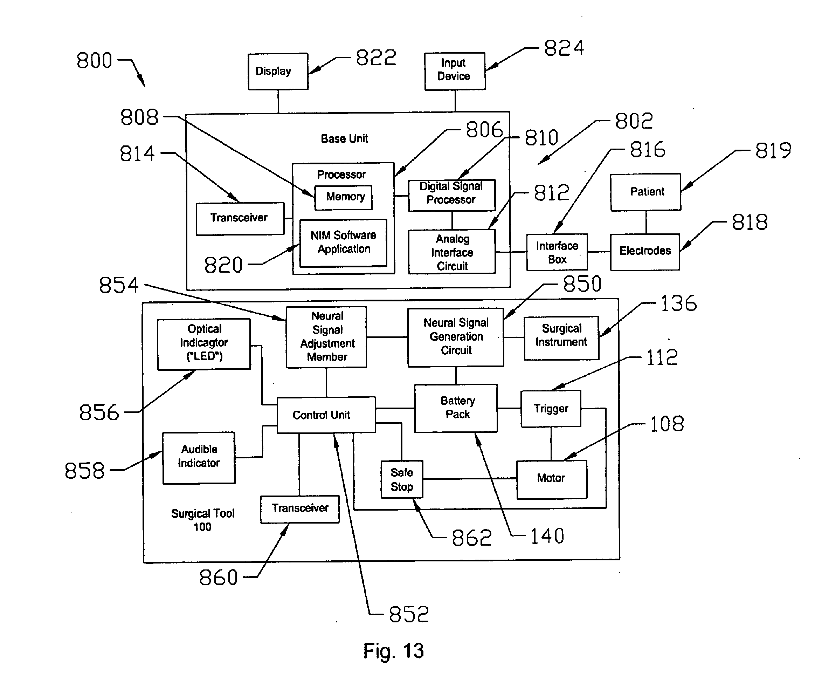

According to another form, surgical tool 100 includes an audible indicator 408 configured to relay a state of the tool to the user. In this form, audible indicator 408 comprises a speaker connected with control unit 180 (see Fig. 3). As set forth in detail below, audible indicator 408 is capable of generating audible warnings and indications during a surgical procedure. Further, surgical tool 100 can also include a third optical indicator 410 connected with control unit 180. As set forth in greater detail below, optical indicator 410 is capable of generating optical warnings to a surgeon during a surgical procedure. In another for, as set forth in greater detail below, elements 404, 406 can comprise two buttons that comprise a neural signal adjustment member 854 (see Fig. 13) used to adjust the intensity of a neural stimulation signal supplied to surgical instrument 136.

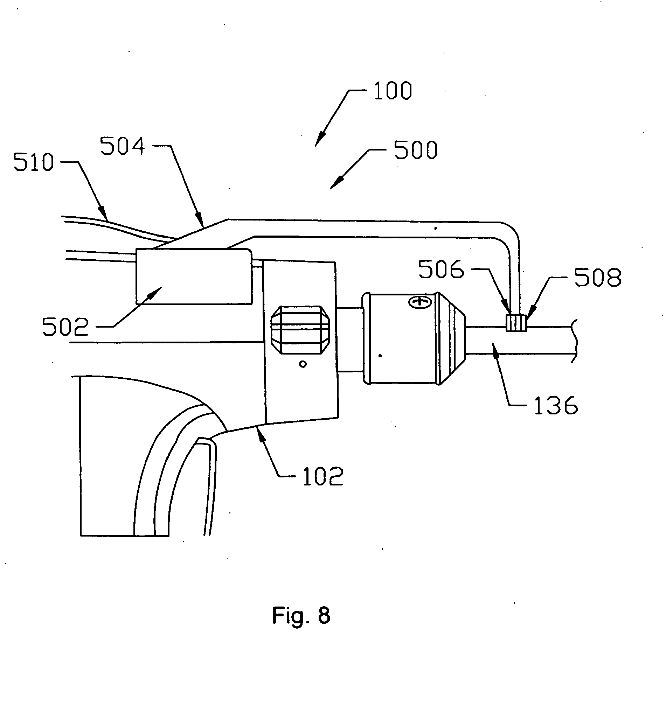

Figs. 8-12 provide illustrations of tool 100 as described herein combined with a neural integrity monitoring (NIM) system 802 (see Fig. 13). Generally, NIM systems are useful for monitoring the neurological status of a patient during particular surgical procedures. For example, during spinal surgeries where the spinal cord, a critical component of the neurological system, is within close proximity of the surgical site, use of a NIM system allows a surgeon to monitor changes and avoid damage to the neurological systems that may occur due to certain surgical procedures. For example, in one particular instance, a surgeon may wish to place anchors within the pedicles of the spinal column for a certain implant. The placement of these anchors must be precise, such that they do not interfere with the spinal cord and, accordingly, integration of a NIM system within the operating room allows a surgeon to monitor the neurological status of the patient while placing the anchors in the patient, providing the surgeon with greater assurance that the placed anchors do not adversely affect the neurological system of the patient.

As set forth in detail below, NIM systems can have a variety of components, but generally include a series of electrodes placed on the patient during surgery, a tool capable of carrying an electrical signal, and a monitor capable of receiving the electrical signals from the tool and monitoring the neurological status of the patient. As will be illustrated in Figs. 8-14, given the design of surgical tool 100 and its intended use, particular designs of NIM system integrations are particularly designed to integrate with surgical tool 100.

Fig. 8 includes a side view of a portion of surgical tool 100 and a NIM member connected to surgical tool 100. As illustrated in Fig. 8, a NIM member 500 includes a base assembly 502 connected to housing 102 of surgical tool 100. In accordance with one form, NIM member 500 further includes an arm 504 extending from base assembly 502 and extending over housing 102 of surgical tool 100. As set forth in greater detail below, in one particular form, base assembly 502 contains a neural signal generation circuit 850 (see e.g. Fig. 13) operable to generate an expected electrical neural stimulation signal. In this form, electrical power utilized to form the neural stimulation signal is provided by battery pack 140 or alternatively, NIM system 802.

Arm 504 includes an active assembly 506 at the distal end of arm 504 configured to make electrical contact with an electrically conductive portion of surgical instrument 136. Generally, when using a NIM system 802 (see Fig. 13), portions of the tools, such as surgical instrument 136 and NIM member 500, in this example, have electrically conductive portions facilitating the transmission of electrical signals between surgical tool 100 and a NIM system 802 (described in detail below). Accordingly, reference to an active assembly as used herein includes a portion of NIM member 500 that is capable of transmitting the expected electrical neural stimulation signal to surgical instrument 136.

Surgical instrument 136 illustrated in Fig. 8 can have a conductive portion facilitating transmission of a signal from surgical tool 100 to a neurological structure, which can then be sensed by NIM system 802. In accordance with this form, active assembly 506 includes a brush 508 having at least one electrically conductive bristle, or possibly a plurality of electrically conductive bristles, electrically connected to the conductive portion of surgical instrument 136. The electrical contact of brush 508 with the conductive portion of surgical instrument 136 facilitates transmission of the neurological electric stimulation signal from active assembly 502 of NIM member 500 to surgical instrument 136. In accordance with one form, active assembly 502 can include power driven components and/or circuits (see e.g. - neural signal generation circuit 850) that can be connected to a power supply. In a more particular form, active assembly 502 can include components or circuits that are electrically connected and powered by battery pack 140 contained within surgical tool 100.

Fig. 8 further illustrates a wire 510 extending from arm 504. In accordance with a particular form, arm 504 has an electrically conductive portion that can electrically connect wire 510 with active assembly 502 for transmission of an electrical signal there through. Wire 510 can connect NIM member 500 with a NIM system 802 within the operating room. As will be described in more detail with other particular forms, NIM member 500 or surgical tool 100 can alternatively have wireless capabilities such that it can wirelessly transmit and receive signals to and from NIM system 802. Moreover, it will be appreciated that for all NIM member embodiments described herein, a wire connection or wireless connection can be utilized for transmitting a signal between the active assembly and NIM system 802.

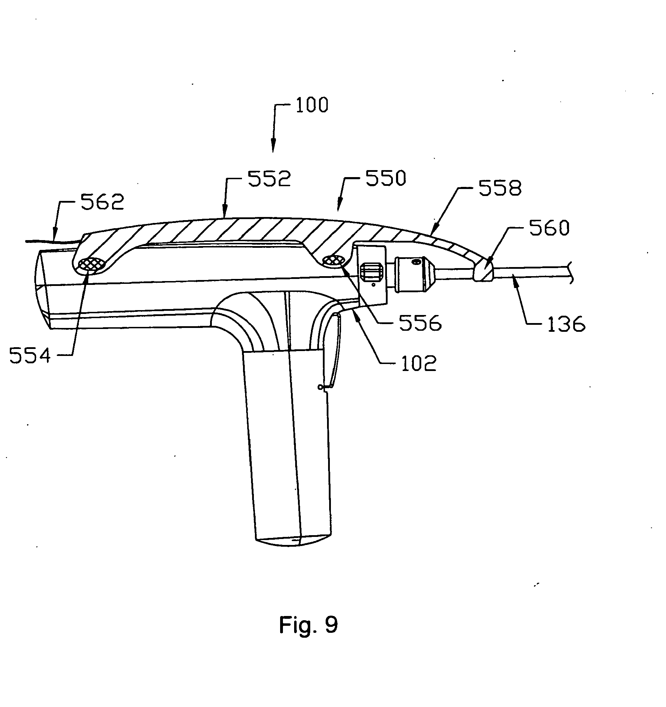

Fig. 9 illustrates a side view of a surgical tool 100 and a NIM member 550 connected in accordance with another representative form. As illustrated, NIM member 550 includes a base assembly 552 coupled to housing 102 of surgical tool 100 via couplings 554 and 556. Base assembly 552 extends along the majority of the length of housing 102 and has a particularly low profile designed to reduce interference with line-of-sight operations of the surgeon. In one form, couplings 554, 556 can connect to housing 102 via a snap-fit or friction fit connection. In another form, couplings 554, 556 are connected to housing 102 via magnets. Use of such couplings 554, 556 facilitates selective coupling and decoupling of NIM member 550 with surgical tool 100.

As illustrated, NIM member 550 further includes an arm 558 extending from base assembly 552 over housing 102 and the quick-connect coupling. NIM member 550 further includes an active assembly 560 coupled to the distal end of arm 558 and configured to electrically connect active assembly 560 with a conductive portion of surgical instrument 136. In accordance with a particular form, active assembly 560 can be connected to surgical instrument 136 such that it substantially surrounds the circumference of surgical instrument 136. In accordance with an alternative form, active assembly 560 can further include a slip-ring contact with surgical tool 100 configured to make electrical contact with the conductive portion of surgical instrument 136. NIM member 550 further includes a wire 562 extending from base assembly 552 and configured to provide an electrical connection and transmission of signals to and from NIM system 802. As described in detail below, in alternative forms, surgical tool 100 is configured to communicate wirelessly with NIM system 802.

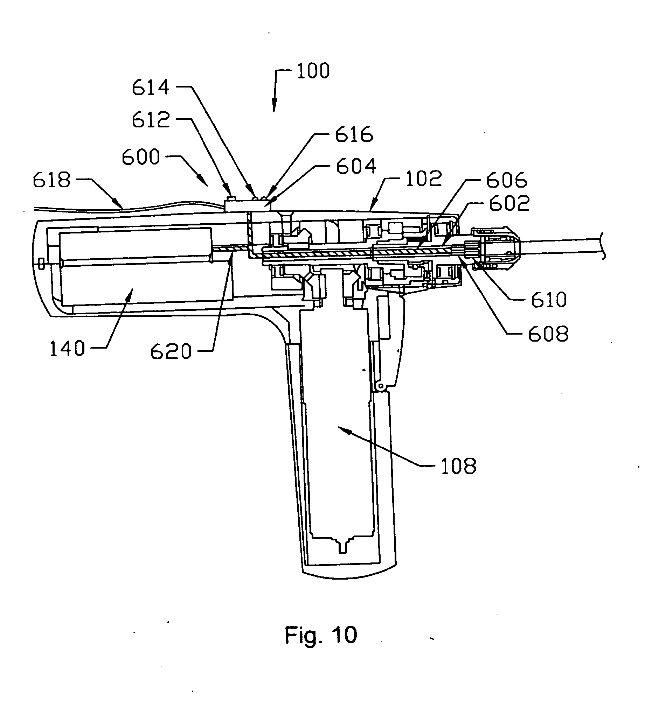

Fig. 10 illustrates a cross-sectional view of a portion of surgical tool 100 including a NIM member 600 in accordance with yet another representative form. As previously described herein, surgical tool 100 can include a passage 602 generally extending from a distal end to a proximal end of housing 102 and more particularly extending through battery pack 140. NIM member 600 includes a base assembly 604 connected to housing 102. Base assembly 604 includes a conductive portion 606 extending within the interior of surgical tool 100 and more particularly extending along at least a portion of passage 602 within the interior of surgical tool 100. As set forth in greater detail below, base assembly 604 includes a neural signal generation circuit or unit 850 (see Fig. 13) that is operable to generate an expected neural stimulation signal. In one form, the expected neural stimulation signal is generated from electrical power provided by battery pack 140, but in alternative forms can be generated by NIM system 802 and transmitted to base assembly 604 via wires 618.

In this form, NIM member 600 includes an active assembly 608 connected to conductive portion 606 and disposed within a portion of passage 602. Active assembly 608 electrically connects conductive portion 606 to a conductive portion of surgical instrument 136, thereby allowing transmission of the expected neural stimulation signal generated by neural signal generation circuit 850 to surgical instrument 136. In a more particular form, active assembly 608 is disposed within a portion of passage 602 abutting a quick-connect coupling 610, such that upon engagement of surgical instrument 136 within quick-connect coupling 610 an electrical connection is formed between active assembly 608 and a conductive portion of surgical instrument 136.

In forms utilizing a conductive portion 606 disposed within a portion of passage 602, at least a portion of passage 602 can include an electrically insulating liner material to avoid electrical connection of conductive portion 606 with other electrical components within the interior of surgical tool 100. Moreover, an electrical liner may provide suitable electromagnetic shielding between conductive portion 606 and active assembly 608 with motor 108 within the housing 102. Generally, at least the portion of passage 602 housing conductive portion 606 comprises the electrically insulating liner. However, in accordance with a more particular form, the entire length of passage 602 may include an electrically insulating liner. Still, in another form, conductive portion 606 can include an electrically insulating portion, such as a liner wrapping around the exterior for further electrical insulation.

Base assembly 604 can include a switch 612 operable between an on state and an off stated for selective operation of NIM member 600. In other forms, switch 612 can comprise an adjustment member allowing the surgeon to increase or decrease the current intensity of the expected neural stimulation signal. Base assembly 604 can further include indicators 614 and 616, such as audible indicators or optical indicators, suitable for notifying the surgeon of certain neurological conditions of the patient. For example, indicator 614 can be an LED providing one indication when the condition of the patient is normal and, alternatively, indicator 616 can be an LED providing a different indication when the condition of the patient has changed or is abnormal. Accordingly, the indicators 614, 616 can aid the surgeon in carrying out a precise and safe surgical procedure.

As further illustrated, NIM member 600 further includes a wire 618 extending from base assembly 604 and configured to transmit electrical signals between active assembly 608 and NIM system 802 within the operating room. Moreover, in yet another form, base assembly 604 can be connected to a power source. In accordance with one particular form, base assembly 604 can be electrically connected to battery pack 140 via an electrical connection 620. It will be appreciated that while the electrical connection between base assembly 604 and battery pack 140 may also include extension of an electrical connection 620 within the passage 602. As previously set forth, base assembly 604 includes a neural signal generation circuit 850 that is capable of generating an expected neural stimulation signal that is applied to surgical tool 136.

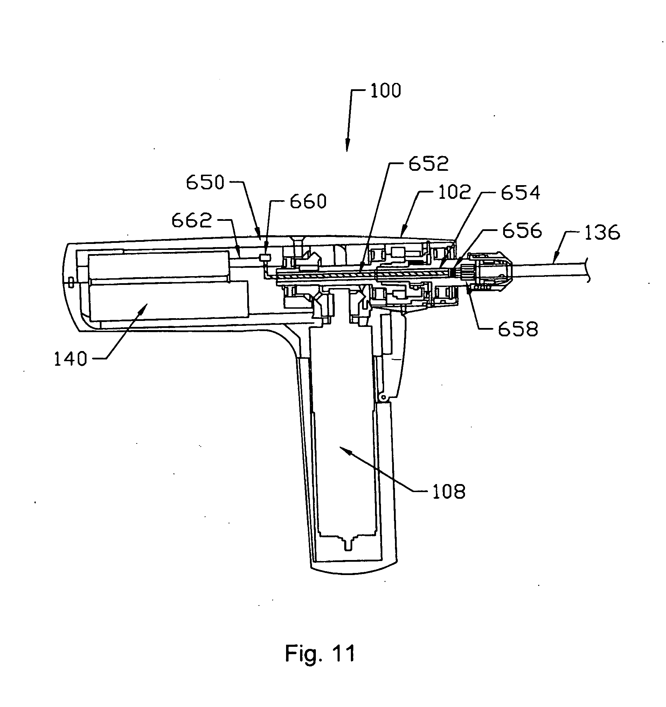

Fig. 11 illustrates a cross-section illustration of surgical tool 100 incorporating NIM member 650 in accordance with yet another form. As illustrated, NIM member 650 includes a conductive portion 652 disclosed within a portion of a passage 654 within the interior of housing 102. As further illustrated, NIM member 650 further includes an active assembly 656 electrically connected to conductive portion 652 and disposed within a portion of passage 654 proximate to a quick-connect coupling 658 and configured to connect active assembly 656 with a conductive portion of surgical instrument 136.

In this particular form, electrically conductive portion 652 is electrically connected to a control assembly 660 disposed within the interior of housing 102 of surgical tool 100. Control assembly 660 is configured to generate an expected neural stimulation signal that is transmitted to conductive portion 652 and thereby to active assembly 654. Control assembly 660 is also configured to transmit and receive signals wirelessly between control assembly 660 and NIM system 802. Control assembly 660 can be connected to a power source, and more particularly, connected to battery pack 140 via an electrical connection 662. While control assembly 660 is illustrated as being disposed within housing 102, in another suitable form, control assembly 660 is connected to housing 102 in an external configuration.

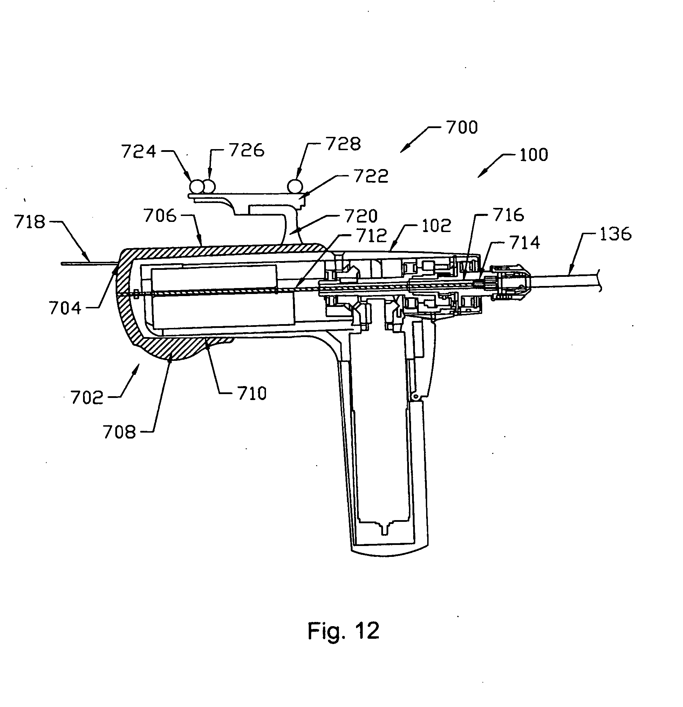

Fig. 12 illustrates a cross-sectional illustration of surgical tool 100 incorporating a navigation enabling member 700 and a NIM member 702. As illustrated, NIM member 702 includes a base assembly 704 having an upper arm 706 and a lower arm 708 connected to housing 102 of surgical tool 100. Arm 708 extends along the lower portion of housing 102 and includes a flange 710 configured to engage a coupling portion or depression within housing 102 and fixably attach base assembly 704 to housing 102. NIM member 702 further includes a conductive portion 712 and active assembly 714 extending the length of a passage 716 from the proximal end of housing 102 to the distal end of the housing 102. NIM member 702 further includes a wire 718 extending from base assembly 704 and configured to electrically connect active assembly 714 with NIM system 802.

As further illustrated, surgical tool 100 can include navigation enabling member 700 connected to base assembly 704 and more particularly, the upper arm 706 of NIM member 702. In accordance with this form, navigation enabling member 700 includes a base 720 directly connected to arm 706. Navigation enabling member 700 further includes an upper portion 722 supporting reflecting structures 724, 726 and 728 that are configured to reflect infrared light emitted by a detector and facilitate triangulation of tool 100 within the operating room. The combination of NIM member 702 and navigation enabling member 700 facilitates improved computer assisted surgeries wherein the surgeon is capable of locating and orienting surgical tool 100 within the operating room as well as monitoring the neural status of the patient during delicate procedures such as spinal surgeries. As with previous forms, NIM member 702 can include a neural signal generation circuit 850 connected with conductive portion 712 for transmitting an expected neural stimulation signal to surgical instrument 136.

Referring to Fig. 13, a system 800 is illustrated that wirelessly integrates surgical tool 100 with NIM system 802. NIM system 802 includes a base unit 804 having a processor 806, memory 808, a digital signal processor 810, an analog interface circuit 812, and a transceiver or receiver 814. In this form, an interface box 816 is connected with analog interface circuit 812. As illustrated, a plurality of electrodes 818 is connected with interface box 816. In other forms, electrodes 818 can connect directly to NIM system 802 and in particular, can connect directly to analog interface circuit 812. In one form, analog interface circuit 812 comprises an analog to digital converter that is capable of converting analog input signals received from electrodes 818 into digital signals.

During a surgical procedure, electrodes 818 are connected to a patient 819 at various locations to monitor neurological responses of the patient during surgery. In one form, electrodes 818 provide electromyographic ("EMG") readings, in the form of muscular responses from nerve stimulation by the neural stimulation signal provided by surgical instrument 136, to NIM system 802. In this form, once electrodes 818 have been connected to the patient at various locations on the body of the patient, electrodes 818 are connected to interface box 816. Electrodes 818 typically include a plurality of sensing electrodes and one or more grounding electrodes. Interface box 816 provides an electrical interface between electrodes 818 and base unit 804. In particular, electrodes 818 transfer electric signals, corresponding to sensed neurological responses of the patient, to interface box 816 for processing by NIM system 802.

As previously set forth, surgical instrument 136 of surgical tool 100 is provided with an expected neural stimulation signal from a neural signal generation circuit 850. In the case of a spinal procedure, for example, if surgical instrument 136 travels within a predetermined proximity of a nerve of the patient or if surgical instrument 136 is in contact with an implant 73, a neurological response is generated as a function of the expected electric signal that can be detected by one or more electrodes 818. Since surgical instrument 136 is electrically conductive as well as implant 73, the expected neural stimulation signal can be detected by a respective neural element near either surgical instrument 136 and/or implant 73. Surgical instrument 136 thus transmits the neural stimulation signal to implant 73 because surgical instrument 136 is in physical contact with implant 73. The response that is detected by NIM system 802 is in the form of an electric signal that is sensed by one or more electrodes 818. This signal is transmitted to interface box 816, which in turn, transmits the detected response to analog interface circuit 812 of NIM system 802.

In one form, analog interface circuit 812 conditions and transmits the sensed signal to digital signal processor 810 that is operable to convert the sensed response into a digital signal that is then transmitted to processor 806. In another form, analog interface circuit 812 comprises an analog to digital circuit that converts the analog response into a digital signal that is transmitted to digital signal processor 806 for further processing. In either form, the response, in the form of a digital signal, is forwarded to processor 806 so that processor 806 can take appropriate action with respect to the received response.

A NIM software application 820 operating on processor 806 is configured to process the digital signal and, amongst other things, generate a graphical indication of the sensed signal on a display 822 connected with base unit 804. As such, because surgical instrument 136 of surgical tool 100 carries an electric signal, surgical tool 100 is capable of generating neurological responses during a surgical procedure if surgical instrument 136 or implant 73 is positioned within a predetermined distance of a neural element. In the case of spinal surgeries, as set forth below, this allows a surgeon to precisely determine and take appropriate action to avoid potentially harmful contact with neural elements.

As further illustrated in Fig. 13, in this form, surgical tool 100 includes a control unit 852, a neural signal adjustment member 854, an optical indicator 856, an audible indicator 858, and a transceiver 860. Further, in one form, surgical tool 100 includes a safe stop circuit 862 connected with control unit 852 and motor 108 that is configured and operable to stop motor 108. In alternative forms, control unit 852 can be connected directly to motor 108 thereby eliminating the need for safe stop circuit 862 as control unit 852 can be connected with motor 108 and configured and operable to stop motor 108. As set forth in detail below, surgical tool 100 is configured with a safe stop feature that allows NIM system 802 to send a signal to surgical tool 100 that will stop motor 108, thereby stopping rotation of surgical instrument 136, if NIM system 802 detects a neurological response indicating that either surgical instrument 136 and/or implant 73 are too close or nearing a respective neural element.

As set forth above, in one form surgical tool 100 includes a control unit 852. Control unit 852 can comprise a microprocessor-based unit, an application specific integrated circuit ("ASIC"), a combination of analog and digital circuitry, or a combination of any of the aforementioned items. Further, although some components of surgical tool 100 are illustrated as being separate components, it is envisioned that one or more components could be integrated into or be a part of control unit 852. For example, control unit 852 can include a digital to analog circuit configured to operate as a neural signal generation circuit 850 or could include a built-in transceiver 860.

Neural signal generation circuit 850 is connected with control unit 852. Neural signal generation circuit 850 is configured and operable to generate an expected neural stimulation signal that has a predetermined amplitude, pulse and duration as required by NIM system 802. In one form, neural signal generation circuit 850 receives current from battery pack 140 and converts it to the expected neural stimulation signal. The signal is then sent to surgical instrument 136 using one or more of the methods previously set forth. Since surgical instrument 136 is electrically conductive, the signal will inherently transfer to the tip of surgical instrument 136. During a surgical procedure in which implant 73 is being inserted into a bone structure surrounded by neural structures or elements, such as a bone screw for example, when the tip of surgical instrument 136 contacts implant 73, the signal is transferred to at least a portion of implant 73 because implant 73 is electrically conductive as well. As such, surgical instrument 136 and implant 73 are both carriers of the neural stimulation signal.

As illustrated in Fig. 13, surgical tool 100 includes a neural signal adjustment member 854 that is capable of increasing or decreasing the current or intensity of the neural stimulation signal. In one form, the neural stimulation signal is modifiable by two different means. As previously set forth with respect to Fig. 7, surgical tool 100 may include two push buttons 404, 406 on an outside surface of housing 102. Touching a first button 404 will increase the current of the neural stimulation signal and touching a second button 406 will decrease the current of the neural stimulation signal. As such, the first option to adjust the intensity of the neural stimulation signal is on surgical tool 100. Upon adjustment of the value of the neural stimulation signal using buttons 404, 406, in one form, surgical tool 100 sends a wireless signal to NIM system 802 to inform NIM system 802 of the updated properties of the neural stimulation signal.

As previously set forth, in this form surgical tool 100 includes a transceiver 860 and NIM system 802 includes a second transceiver 814. Transceivers 814, 860 are operable to transmit and receive radio signals such that surgical tool 100 and NIM system 802 are capable of conducting wireless communication with each other. NIM system 802 can include an input device 824 that allows a user to select and modify the intensity of the neural stimulation signal. In response, NIM software application 820 instructs transceiver 814 to transmit a wireless signal to surgical tool 100 instructing surgical tool 100 to either increase or decrease the current of the neural stimulation signal. Transceiver 860 of surgical tool 100 receives this signal from NIM system 802 and in response, control unit 852 instructs or causes neural signal generation circuit 850 to either increase or decrease the current of the neural stimulation signal accordingly. As such, in this form, NIM system 802 is capable of wirelessly controlling the current or relevant variables of the neural stimulation signal. The new properties of the neural stimulation signal are stored in memory 808 and used by NIM software application 820 to determine what type of response to look for from electrodes 818.

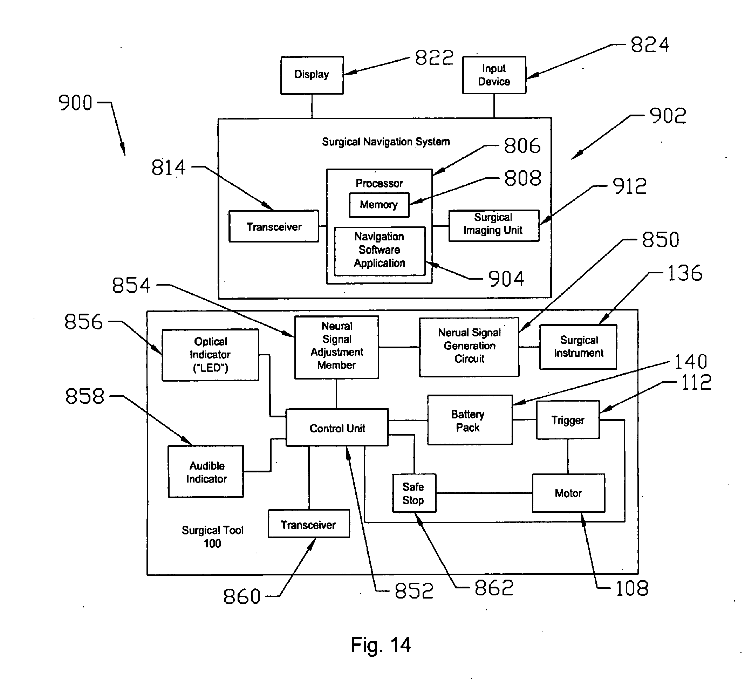

Surgical tool 100 and NIM system 802 are configured to communicate with one another for a variety of reasons. As set forth above, in one form surgical tool 100 is configured to communicate an adjustment of the neural stimulation signal via push buttons 404, 406 to ensure that the signal (i.e. - current, duration, pulse, frequency, and so forth) that is directed to surgical instrument 136 is the same as that expected by NIM system 802. Another reason, as described in detail below, is to be able to relay the neurological status of patient 819 to surgical tool 100 which will allow optical indicator 856 to change colors as appropriate and to provide a safe stop feature. Yet another is to provide a means to communicate with a navigation system 902 (see Fig. 14) for another type of safe stop feature.

As briefly set forth above, surgical tool 100 is configured with one or more safe stop features that automatically disengage motor 108 to ensure that surgical tool 100 and/or implant 73 are not positioned at a point that would cause neurological complications in patient 819. In one form, if a response or signal is received from one or more electrodes 818 by NIM system 802 above a specified neural threshold or value, which is determined, amongst other things, as a function of the properties of the neural stimulation signal, NIM software application 820 will instruct or control transceiver 814 to send a wireless signal to surgical tool 100 that will cause surgical tool 100 to stop motor 108. As illustrated, in this form, trigger mechanism 112 is connected with battery pack 140 is connected between battery pack 140 and motor 108. Further, trigger mechanism 112 is connected with control unit 852.

During normal operation, when trigger mechanism 112 is depressed, current passes from battery pack 140 to motor 108 thereby allowing motor 108 to rotate surgical instrument 136. If a response signal is received by NIM system 802 that is above the predefined threshold, a wireless signal is sent by NIM system 802 to surgical tool 100 that causes control unit 852 to send a signal to trigger mechanism 112 that causes trigger mechanism 112 to cease supplying current to motor 108 thereby causing surgical instrument 136 to cease rotating. In the alternative, a safe stop circuit 862 can be connected with control unit 852 and motor 108. In this form, control unit 852 causes safe stop circuit 862 to force motor 108 to shut down. If the safe stop feature is triggered, it can be reset either on NIM system 802 or by waiting a specified amount of time (e.g. - five seconds).

Surgical tool 100 can further include optical indicator 856 that is connected with control unit 852 and located on an outer surface of housing 102 in a readily visible location of the operator. In one form, optical indicator 856 comprises a light emitting diode ("LED") that is capable of changing between two or more colors (e.g. - red and greed). If no response is detected by NIM system 802 or navigation system 902, optical indicator 856 will be green. If a response is detected that indicates that the neurological status of patient 819 is potentially at risk, control unit 852 is operable to cause optical indicator 856 to light up red to indicate to the operator that a safe threshold has been reached or of the potential risk.

In yet another form, surgical tool 100 can also include an audible indictor 858 that is capable of audibly notifying the operator of the potential risk by generating an audible noise that can be heard by the operator. NIM software application 820 further includes a software application configured to operate NIM system 802 in a nerve proximity mode that causes surgical tool 100 to operate in a predefined manner. In the nerve proximity mode, the audible noises generated by surgical tool 100 correspond to how much current is being sensed by the neural element. As such, the closer surgical instrument 136 and/or implant 73 get to a respective neural element, the louder and more frequent the audible noises become. This is because the strength of the response sensed by NIM system 802 may not have reached the specified threshold, thereby requiring the safe stop feature to be initiated, but yet it still may be important for the operator to be informed that a potential risk is approaching. As such, NIM system 802 sends a plurality of wireless signals to surgical instrument 100 instructing control unit 852 to increase the volume, frequency and/or pitch of the audible noise as the strength of the response increases (i.e. - the closer surgical instrument 136 and/or implant 73 get to a respective neural element). Once the response received by NIM system 802 reaches the specified threshold, NIM system 802 sends a wireless signal to surgical tool 100 that causes motor 108 to shut down (i.e. - initiates the safe stop feature).

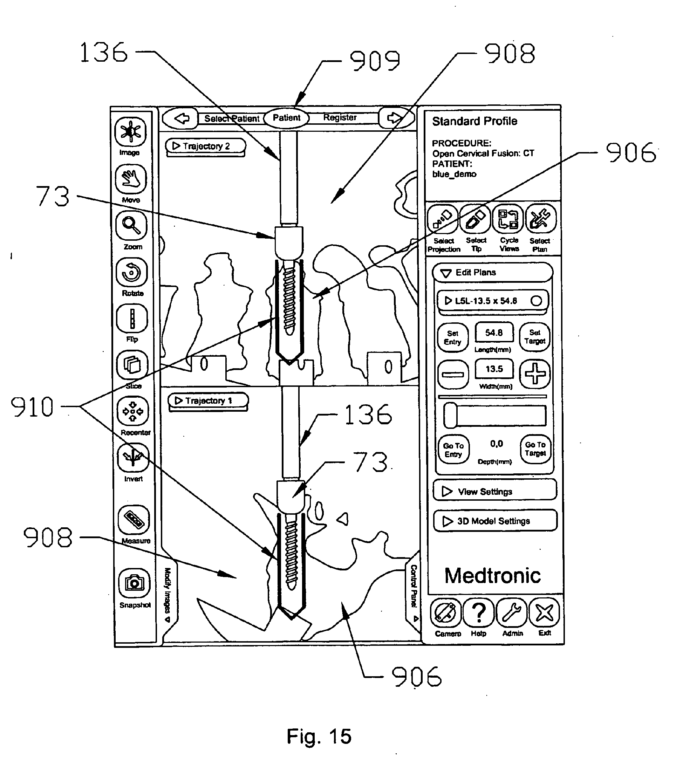

Referring to Figs. 14 and 15, wherein like numerals correspond to common elements found in Fig. 13, a system 900 is illustrated that integrates surgical tool 100 with a surgical navigation ("NAV") system 902. As previously set forth with respect to the surgical tool 100 discussed with respect to Fig. 12, in some forms, surgical tool 100 can include a navigation enabling member 700 that allows NAV system 902 to determine the relative position of surgical tool 100 as it relates to the patient. As such, as surgical tool 100 is moved relative to the patient, a navigation software application 904 located on NAV system 902 is capable of determining the relative position or location of surgical tool 100 in relation to a bone structure 906 of the patient that will receive implant 73.

As illustrated in Fig. 15, prior to beginning a surgical procedure, NAV system 902 will be used to generate one or more radiographic images 908 of bone structure 906. These radiographic images 908 can be used to generate three-dimensional views of bone structure 906 that can be viewed on display 822. As such, NAV software application 904 includes one or more software applications that are configured and operable to generate a graphical user interface 909 on display 908. NAV system 902 includes, or in the alternative is connected with, a surgical imaging unit 912 that is configured and operable to generate images 908. In addition to generating images 908 of bone structure 906, navigation software application 904 includes software applications configured and operable to determine the physical location of bone structure 906 in the operating room. As such, NAV system 902 knows the physical location of surgical tool 100 and bone structure 906.

Navigation software application 904 further includes one or more software applications configured and operable to allow an operator to use input device 824 to define one or more safe or target thresholds 910 on images 908. These safe or target thresholds 910 on images 908 correspond to the location in bone structure 906 in which implant 73 is to be implanted into bone structure 906 in a position desired by the surgeon as well as in a position that will avoid affecting the neural integrity of the patient. Since surgical instrument 136 and implant 73 can be viewed on radiographic images 908, navigation software application 904 further includes software applications configured and operable to generate updated images 908 during the surgical procedure to help the surgeon monitor the progress of the placement of implant 73.

As set forth above, safe or target thresholds 910 are defined on images 908 using NAV system 902. During implantation of implant 73 into bone structure 906, if a safe threshold 910 is breached or about to be breached, navigation software application 904 will instruct transceiver 814 to transmit a wireless signal to surgical tool 100 instructing surgical tool 100 to stop. As such, NAV system 902 is capable of transmitting a wireless signal to surgical tool 100 during the surgical procedure that will stop motor 108. As with the previous form, NAV system 902 can be utilized to reset surgical tool 100 after it has been stopped or surgical tool 100 can be configured to reset after a specified amount of time (e.g. five seconds). As such, surgical tool 100 is provided with a safe stop feature for use in conjunction with NAV system 902.

In yet another form, surgical tool 100 includes optical indicator 856 that control unit 852 uses to create a visual notification to the operator. In particular, if safe threshold 910 has not been breached, optical indicator 856 will light up green. If safe threshold 910 has been breached or is about to be breached, NAV system 902 can transmit a wireless signal to surgical tool 100 instructing optical indicator 856 to light up red. As a result of receipt of this wireless signal, control unit 852 causes optical indicator 856 to change colors.

In another form, surgical tool 100 includes audible indicator 858 that is configured and operable by control unit 852 to operate in a proximity beeping mode. As implant 73 approaches safe threshold 910, NAV system 902 instructs transceiver 814 to transmit a wireless signal to surgical tool 100 that indicates to control unit 852 that implant 73 is approaching safe threshold 910. As a result of receipt of this signal by transceiver 860, control unit 852 changes the volume, frequency and/or pitch of an audible noise being generated using audible indicator 858. For example, the audible noise generated using audible indicator 858 can get louder and faster the closer implant 73 gets to safe threshold 910.

Although the preferred embodiments have been depicted and described in detail herein, it will be apparent to those skilled in the art that various modifications, additions and substitutions can be made without departing from its essence and therefore these are to be considered to be within the scope of the following claims.

## Claims

1. A combination of a surgical tool (100) with a neural integrity monitoring system (802) and with an implant (73),
the neural integrity monitoring system (802) being operable to monitor a neurological status of a patient and having a neural signal generation unit (850) for generating an expected signal;
the surgical tool (100) comprising a housing (102), a motor (108) disposed within the housing (102) and coupled to an output shaft (120), a surgical instrument (136) connected with the output shaft (120) to be rotated by engagement of the motor (108) and having a tip for contacting the implant (73), a trigger mechanism configured to selectively supply power to said motor (108) to rotate said output shaft (120), and a neural integrity monitoring member (500, 600, 702) for providing the expected signal, generated by the neural signal generation unit (850), to the surgical instrument (136),
wherein both the surgical instrument (136) and the implant (73) are electrically conductive so that, when the tip of the surgical instrument contacts the implant (73), the surgical instrument (136) and the implant (73) are both carriers of the neural stimulation signal.

2. The combination of claim 1, wherein the surgical tool (100) further comprises a receiver for receiving wireless communication signals, and wherein the neural integrity monitoring system (802) wirelessly is operable to transmit a motor shut down signal to said surgical tool (100) if said neural integrity monitoring system (800) determines that said neurological status of said patient is at risk as a function of a response received to said expected electric signal.

3. The combination of claim 2, wherein the surgical tool (100) further comprises an adjustment mechanism connected with said neural signal generation unit (850) configured to allow adjustment of said expected electrical signal.

4. The combination of claim 2, wherein said surgical tool (100) further comprises an optical indicator located on an outside surface of said housing (102) configured to generate a visual indication indicative of a neurological status of said patient.

5. The combination of claim 2, wherein said surgical tool (100) further comprises an audible indicator configured to generate an audible alarm if said response received by said neural integrity monitoring system (802) is above a specified threshold.

6. The combination of claim 5, wherein said integrity monitoring system (802) is configured to operate in a nerve proximity mode prior to receiving a respective response above said specified threshold.

7. The combination of claim 6, wherein said nerve proximity mode is configured to cause said surgical tool (100) to generate audible responses that increase in intensity as a function of a sensed response of said expected electric signal by said neural integrity monitoring system (802).

## Patentansprüche

1. Eine Kombination eines chirurgischen Werkzeugs (100) mit einem Neurale-Unversehrtheit-überwachungssystem (802) und mit einem Implantat (73),
wobei das Neurale-Unversehrtheit-Überwachungssystem (802) betätigbar ist, um einen neurologischen Zustand eines Patienten zu überwachen, und eine Neuralsignal-Erzeugungseinheit (850) aufweist, um ein erwartetes Signal zu erzeugen,
wobei das chirurgische Werkzeug (100) aufweist: ein Gehäuse (102), einen Motor (108), der innerhalb des Gehäuses (102) angeordnet ist und mit einer Ausgangswelle (120) verbunden ist, ein chirurgisches Instrument (136), das mit der Ausgangswelle (120) verbunden ist, um durch einen Eingriff des Motors (108) gedreht zu werden, und eine Spitze zum Kontaktieren des Implantats (73) aufweist, einen Auslösemechanismus, der eingerichtet ist, um dem Motor (108) wahlweise Energie zuzuführen, um die Ausgangswelle (120) zu drehen, und ein Neurale-Unversehrtheit-Überwachungselement (500, 600, 702), um das von der Neuralsignal-Erzeugungseinheit (850) erzeugte erwartete Signal an das chirurgische Instrument (136) zuzuführen,
wobei sowohl das chirurgische Instrument (136) als auch das Implantat (73) elektrisch leitfähig sind, so dass, wenn die Spitze des.chirurgischen Instruments das Implantat (73) kontaktiert, das chirurgische Instrument (136) und das Implantat (73) beide Träger des Neuralstimulationssignals sind.

2. Die Kombination gemäß Anspruch 1, wobei das chirurgische Werkzeug (100) ferner einen Empfänger aufweist, um drahtlose Kommunikationssignale zu empfangen, und wobei das Neurale-Unversehrtheit-Überwachungssystem (802) drahtlos betätigbar ist, um ein Motor-Abschaltsignal an das chirurgische Werkzeug (100) zu übermitteln, wenn das Neurale-Unversehrtheit-Überwachungssystem (800) ermittelt, dass der neurologische Zustand des Patienten gefährdet ist, in Abhängigkeit von einer auf das erwartete elektrische Signal empfangenen Antwort.

3. Die Kombination gemäß Anspruch 2, wobei das chirurgische Werkzeug (100) ferner einen mit der Neuralsignal-Erzeugungseinheit (850) verbundenen Anpassungsmechanismus aufweist, der eingerichtet ist, um ein Anpassen des erwarteten elektrischen Signals zu ermöglichen.

4. Die Kombination gemäß Anspruch 2, wobei das chirurgische Werkzeug (100) ferner einen an einer Außenfläche des Gehäuses (102) angeordneten optischen Indikator aufweist, der eingerichtet ist, um eine visuelle Anzeige zu erzeugen, die einen neurologischen Zustand des Patienten anzeigt.

5. Die Kombination gemäß Anspruch 2, wobei das chirurgische Werkzeug (100) ferner einen akustischen Indikator aufweist, der eingerichtet ist, um eine akustische Warnung zu erzeugen, wenn die von dem Neurale-Unversehrtheit-Überwachungssystem (802) empfangene Antwort über einer bestimmten Schwelle liegt.

6. Die Kombination gemäß Anspruch 5, wobei das Unversehrtheit-Überwachungssystem (802) eingerichtet ist, um vor dem Empfangen einer jeweiligen Antwort über der bestimmten Schwelle in einem Nerven-Nähe-Modus zu funktionieren.

7. Die Kombination gemäß Anspruch 6, wobei der Nerven-Nähe-Modus eingerichtet ist, um zu bewirken, dass das chirurgische Werkzeug (100) akustische Antworten erzeugt, deren Stärke in Abhängigkeit von einer erfassten Antwort des erwarteten elektrischen Signals durch das Neurale-Unversehrtheit-Überwachungssystem (802) ansteigt.

## Revendications

1. Combinaison d'un outil chirurgical (100) avec un système de surveillance d'intégrité nerveuse (802) et avec un implant (73),
le système de surveillance d'intégrité nerveuse (802) étant utilisable pour surveiller un état neurologique d'un patient et ayant une unité de génération de signal nerveux (850) pour générer un signal attendu ;
coutil chirurgical (100) comprenant un boîtier (102), un moteur (108) disposé dans le boîtier (102) et couplé à un arbre de sortie (120), un instrument chirurgical (136) relié avec l'arbre de sortie (120) pouvant tourner par mise en prise du moteur (108) et ayant une pointe pour entrer en contact avec l'implant (73), un mécanisme de déclenchement configuré pour fournir de l'énergie de manière sélective audit moteur (108) pour faire tourner ledit arbre de sortie (120), et un élément de surveillance d'intégrité neurale (500, 600, 702) pour fournir le signal attendu, généré par l'unité de génération de signal nerveux (850), à l'instrument chirurgical (136),
où à la fois l'instrument chirurgical (136) et l'implant (73) sont électriquement conducteurs de sorte que, quand la pointe de l'instrument chirurgical entre en contact avec l'implant (73), l'instrument chirurgical (136) et l'implant (73) sont tous les deux porteurs du signal de stimulation nerveuse.

2. Combinaison selon la revendication 1, où l'instrument chirurgical (100) comprend en outre un récepteur pour recevoir des signaux de communication sans fil, et où le système de surveillance d'intégrité nerveuse (802) est utilisable sans fil pour émettre un signal d'arrêt du moteur audit outil chirurgical (100) si ledit système de surveillance d'intégrité nerveuse (800) détermine que ledit état neurologique dudit patient présent un risque en fonction d'une réponse reçue dudit signal électrique attendu.

3. Combinaison selon la revendication 2, où l'outil chirurgical (100) comprend en outre un mécanisme d'ajustement relié avec ladite unité de génération de signal nerveux (850) configuré pour permettre l'ajustement dudit signal électrique attendu.

4. Combinaison selon la revendication 2, où ledit outil chirurgical (100) comprend en outre un indicateur optique situé sur une surface extérieure dudit boîtier (102) configuré pour générer une indication visuelle indicatrice d'un état neurologique dudit patient.

5. Combinaison selon la revendication 2, où ledit outil chirurgical (100) comprend en outre un indicateur sonore configuré pour générer une alarme sonore si ladite réponse reçue par ledit système de surveillance d'intégrité nerveuse (802) est au-dessus d'un palier spécifié.

6. Combinaison selon la revendication 5, où ledit système de surveillance d'intégrité (802) est configuré pour fonctionner dans un mode de proximité nerveuse avant de recevoir une réponse respective au-dessus dudit palier spécifié.

7. Combinaison selon la revendication 6, où ledit mode de proximité nerveuse est configuré pour amener ledit outil chirurgical (100) à générer des réponses sonores qui augmentent en intensité en fonction d'une réponse détectée dudit signal électrique attendu par ledit système de surveillance d'intégrité nerveuse (802).
